# EUROPEAN PATENT APPLICATION

(11) **EP 4 512 314 A1**
(43) Date of publication of application: **26.02.2025**
(21) Application number: 24192447.1
(22) Date of filing: 01.08.2024
(51) Int. Cl.: A61B 5/0205, A61B 5/00, A61B 5/024

(54) **A WEARABLE DEVICE COMPRISING OPTICAL AND TEMPERATURE SENSORS**

(30) Priority: 24.08.2023 EP 23193308
(71) Applicant: Withings, 92130 Issy-Les-Moulineaux (FR)
(72) Inventor: TUCOULAT, Benoît, 92130 Issy-les-Moulineaux (FR); KOCZAN, Xavier, Issy-les-Moulineaux (FR); PIMENTA, Victor, Issy-les-Moulineaux (FR)
(74) Representative: Withings IP

(57) **Abstract**

The invention concerns a wearable device configured to be positioned on a user's wrist and comprising:
- a case back configured to be at least partially in contact with the user's wrist,
- a heat flux sensor (322), configured to measure a heat flux coming from the user's wrist, and
- an optical sensor (310) comprising at least one light emission assembly (312) to emit light and at least one light reception assembly (314) to receive light from the light emission assembly (312).

Positions of the at least one light emission assembly (312) and the at least one light reception assembly (314) form a geometrical shape (GS) on the case back (210), as projected orthogonally on the case back (210).

The heat flux sensor (322) is arranged within the geometrical shape (GS), as projected orthogonally on the case back (210).

## Description

### Field of the invention

The present invention relates to a wearable device. The wearable device is in particular a watch, notably a connected watch, or an activity tracker. By watch or activity tracker, it is meant a portable device whose preferred position is at the wrist ("wrist wearable").

The present invention also relates to the field of so-called hybrid connected watches, i.e. connected watches having a visual appearance closer to conventional mechanical watches, in particular thanks to the presence of a gear train as well as mechanical hands to indicate at least the time (hour hand and minute hand).

The present invention more precisely relates to the measure of physiological parameters of the user wearing the wearable device, in particular the user body temperature.

### Background

It is known to provide a watch comprising a temperature sensor configured to measure a skin temperature of the user. Document JP2004/177286 discloses for example such a device. As another example, illustrated by documents US20220026284 and US2023/085860, Apple^{™} discloses a watch with two thermistors (i.e. temperature sensors) measuring respectively the skin temperature and the outside temperature.

Other connected wearable devices may comprise an optical sensor arranged on the case back of the device. In particular, the optical sensor may be a PPG (photoplethysmography) sensor, to measure the user heart rate and/or the saturation in oxygen of the user blood. For example, document WO2021/204209, in the name of Withings^{™}, describes in detail an optical sensor, which is found on the Withings ScanWatch^{™}.

Recently, it has been proposed wearable devices facing the technical challenge of combining both an optical sensor and a temperature sensor on the same watch.

For example, in document WO2023/140464, Samsung^{™} discloses a watch on which the optical sensor is located on the middle of the case back, while an infrared temperature sensor is represented outside the lens on the case back (referred as 276 on figure 2c of this document). This is the case, for example, with the Samsung Galaxy Watch 5.

However, the body temperature measurement could be further improved. To that end, in document WO2018/114653, GreenTeg^{™} discloses a combination of a heat flux sensor and a temperature sensor to measure more precisely core body temperature.

While the temperature measurement is improved, a heat flux sensor is technically quite different from a conventional temperature sensor and generates a whole new set of integration issues (notably the size of the heat flux sensor and the physical properties pertaining to the environment of the heat flux sensor). These technical specificities need to be considered and such a heat flux sensor cannot be integrated in a watch as a conventional skin temperature sensor.

To address this issue, Corsano^{™} implemented such a heat flux sensor on a tracker and disclosed a watch in which the optical sensor is on one side of the case back and the heat flux sensor is on the other side of the case back. This is the case, for example, with the "CardioWatch 287-1".

However, the inventors have found that such an architecture could be further improved to provide better combined optical and temperature measurements.

### Summary of the disclosure

An aim of the disclosure is to provide a wearable device enabling an efficient and reliable optical measure combined with an advanced body temperature determination using at least a heat flux sensor.

To solve at least one of these issues, the disclosure relates to a wearable device configured to be positioned on a user's wrist, the wearable device comprising: a case back configured to be at least partially in contact with the user's wrist; a heat flux sensor, configured to measure a heat flux coming from the user's wrist; and an optical sensor comprising at least one light emission assembly to emit light and at least one light reception assembly to receive light from the light emission assembly, wherein positions of the at least one light emission assembly and the at least one light reception assembly form a geometrical shape on the case back, as projected orthogonally on the case back, wherein the heat flux sensor is arranged within the geometrical shape, as projected orthogonally on the case back.

Thanks to this device, the wearable device meets the high layout constraints for the optical components of the optical sensor while improving the heat flux sensor sensitivity and limiting the influence of heat generated by the light emission assembly. Indeed, by arranging the heat flux sensor inside the geometrical shape formed by the optical sensor: sufficient room is available for the heat flux sensor (which is bulkier than a temperature sensor), the contact between the heat flux sensor and the user's wrist is improved, and heat influence is homogenized. Moreover, the heat flux sensor is less sensitive to the outside temperature and the measured heat flow is close to that of the skin. Also, this case back arrangement enables the sensors to take a limited spatial area on the case back, enabling to arrange other sensors on the case back, such as an ECG electrode for example.

In an embodiment, each of the at least one light emission assembly is configured to emit light towards each of the at least one light reception assembly.

In an embodiment, each of the at least one light reception assembly is configured to receive light from the or each of the at least one light emission assembly.

In one embodiment, the at least one light emission assembly and the at least one light reception assembly are positioned in a same plane, called sensor plane, and the heat flux sensor is also positioned in the sensor plane.

In one embodiment, the heat flux sensor is arranged at the center of the geometrical shape.

In one embodiment, the device further comprises a skin temperature sensor, to determine a skin temperature of the user, the skin temperature sensor is also located within the geometrical shape, as projected orthogonally on the case back.

In one embodiment, the skin temperature sensor is located outside the geometrical shape.

In one embodiment, at least a light emission assembly and at least a light reception assembly are disposed on either side of the heat flux sensor, as projected orthogonally on the case back.

In one embodiment, the heat flux sensor is arranged at the middle of the case back.

In one embodiment, the geometrical shape is a convex shape.

In one embodiment, the optical sensor comprises several light emission assemblies and several light reception assemblies, the formed geometrical shape being a polygon, the heat flux sensor being within the polygon.

In one embodiment, the optical sensor comprises one light emission assembly and one light reception assembly, the formed geometrical shape being a segment.

In one embodiment, the optical sensor comprises one light emission assembly and two light reception assemblies, the formed geometrical shape being a triangle.

In one embodiment, the optical sensor comprises two light emission assemblies and two light reception assemblies, the formed geometrical shape being a rhombus, preferably a square.

In one embodiment, the optical sensor comprises two light emission assemblies and four light reception assemblies, the formed geometrical shape being a hexagon.

In one embodiment, the distance between each light emission assembly and two of the four light reception assemblies is comprised between 3 mm and 4 mm and the distance between each light emission assembly and the other two of the four light reception assemblies is comprised between 8 mm and 9 mm.

In one embodiment, the device further comprises a sensors printed circuit board comprising a topside and a backside, the backside is oriented towards the case back,

the heat flux sensor, the at least one light emission assembly and the at least one light reception assembly are attached to the backside of the sensors printed circuit board.

In one embodiment, the temperature sensor is also attached to the backside.

In one embodiment, the device comprises a main printed circuit board, connected to the sensors printed circuit board, preferably through a flexible circuit board.

In one embodiment, the light emission assembly comprises a green LED, a red LED and at least an infrared LED, preferably two infrared LED.

In one embodiment, the optical sensor comprises an optical module, the optical module being configured to generate the instructions for the light emission assemblies and to recover the electrical signals from the light reception assemblies.

In one embodiment, the optical module is mounted on the backside of the sensors printed circuit board.

In one embodiment, the device further comprises a core body temperature module configured to receive data from the heat flux sensor and advantageously form the skin temperature sensor to determine a core body temperature of the user.

In one embodiment, the core body temperature module is mounted on the backside of the sensors printed circuit board.

In one embodiment, the heat flux sensor is in the form of a sandwich-like structure comprising multiple layers of different materials.

In one embodiment, the skin temperature sensor is a thermistor sensor or an infrared thermal sensor.

In one embodiment, the optical module is configured to determine a heart rate of the user, the core body temperature module being configured to collect the heart rate determined by the optical module and configured to determine the core body temperature of the user based at least on the electrical signals and on the heart rate.

In one embodiment, the device further comprises an accelerometer, the core body temperature module being configured to collect the acceleration signals delivered by the accelerometer and configured to determine the core body temperature of the user based at least on the electrical signals and on the acceleration signals.

In one embodiment, the core body temperature module collects the electrical signals at a frequency comprised between 0.1 Hz and 0.5 Hz, notably 0.2 Hz.

In one embodiment, the device comprises a spacer with a plurality of cavities, wherein the heat flux sensor and the optical sensor are arranged in respective cavities of the plurality of cavities.

In one embodiment, the case back comprises a lens configured to be in contact with the wrist, and the optical sensor and the heat flux sensor are positioned behind the lens, such as a glass lens.

In one embodiment, the skin temperature sensor is also positioned behind the lens.

In one embodiment, the heat flux sensor is arranged at the center of the lens.

In one embodiment, the case back comprises a surrounding member surrounding the lens.

In one embodiment, the spacer is arranged between the sensors printed circuit board and the lens.

In one embodiment, the spacer comprises at least two layers comprising a rigid layer and a compressible layer, preferably two compressible layers on each side of the rigid layer.

In one embodiment, each of the at least one light emission assembly, the at least one light reception assembly and the heat flux sensor are arranged in respective cavities of the plurality of cavities.

In one embodiment, the skin temperature sensor is arranged in the same cavity as the heat flux sensor.

In one embodiment, one of the cavities is a central cavity, located centrally on the case back as projected orthogonally on the case back, the central cavity having wider dimensions than the heat flux sensor, said central cavity being configured to accommodate the heat flux sensor.

In one embodiment, the central cavity is circular.

In one embodiment, the central cavity comprises a thermal conductive gap filler made from a thermally conductive material and surrounding partially the heat flux sensor.

In one embodiment, the central cavity comprises a thermal conductive gap filler made from a thermally conductive material and surrounding partially the skin temperature sensor.

In one embodiment, the thermally conductive material is a silicone elastomer.

In one embodiment, the thermal conductive gap filler enables to homogenize the heat flux and thus minimize parasitic heat flows.

In one embodiment, the conductivity of the thermal conductive gap filler is comprised between 0.3 W/mK and 10 W/mK, preferably between 0.8 W/mK and 3 W/mK.

In one embodiment, the thermal conductive gap filler is compressed between the sensors printed circuit board and the lens, the thermal conductive gap filler filing all the space of the central cavity.

In one embodiment, the device comprises a thermal exhaust made from a thermally conductive material and arranged on the topside of the sensors printed circuit board.

In one embodiment, the thermally conductive material is a silicone elastomer, for example the same as for the gap filer.

In one embodiment, the device comprises a battery, the thermal exhaust being arranged between the sensors printed circuit board and the battery.

In one embodiment, the sensors printed circuit board comprises at least an insulating slot filled with a thermal insulator, in particular filled with air, and arranged between the heat flux sensor and the optical sensor.

In one embodiment, the insulating slot is a pass-through opening.

In one embodiment, the at least one insulating slot is/are arranged all around the heat flux sensor, and eventually the skin temperature sensor.

In one embodiment, at least one insulating slot is arranged between the heat flux sensor and each light emission assembly.

In one embodiment, at least one insulating slot is arranged between the heat flux sensor and the optical module.

In one embodiment, at least one insulating slot is arranged between the heat flux sensor and the core body temperature module.

In one embodiment, each insulating slot forms a portion of a circle, preferably centered on the center of the case back (or centered on the center of the lens)

In one embodiment, the width of each insulating slot is comprised between 0.1 mm and 1 mm, preferably between 0.3 mm and 0.7 mm.

In one embodiment, the device comprises at least two ECG electrodes and an ECG module, electrically connected to the ECG electrodes and configured to perform an electrocardiogram.

In one embodiment, the case back comprises a first ECG electrode.

In one embodiment, the first ECG electrode at least partially surrounds the lens.

In one embodiment, the device comprises a bezel mounted on the enclosure and made of electrically conductive material, the bezel comprising a second ECG electrode.

In one embodiment, the ECG module is configured to impose a potential on one of the two ECG electrodes and to measure a potential of the other of the two ECG electrodes.

The invention also concerns a wearable device configured to be positioned on a user's wrist, the wearable device comprising: a case back configured to be at least partially in contact with the user's wrist; a heat flux sensor, configured to measure a heat flux coming from the user's wrist; and an optical sensor comprising a plurality of light emission assemblies to emit light and at least one light reception assembly to receive light from the light emission assembly, wherein positions of the plurality of light emission assemblies form a geometrical shape on the case back, as projected orthogonally on the case back, wherein the heat flux sensor is arranged within the geometrical shape, as projected orthogonally on the case back.

The invention also concerns a method of determination of a user's core body temperature carried out by a wearable device according to one of the previous claims, the method comprising:
- measure a heat flux coming from the wrist of the user,
- determination of the user's core body temperature based at least on the measured heat flux.

In one embodiment, the wearable further comprises a skin temperature sensor, the method further comprising:
- measure a skin temperature sensor of the user,
- determination of the user's core body temperature based at least on the measured heat flux and the measured skin temperature.

In one embodiment, the method further comprises:
- light emission by light emission assemblies,
- light reception from the light reception assemblies,
- determination of the user heart rate based on the received light,
- determination of the user's core body temperature based at least on the measured heat flux and the user's heart rate.

In one embodiment, the device further comprises an accelerometer, the method further comprising:
- measure an acceleration of the device,
- determination of the user's core body temperature based at least on the measured heat flux and the measured acceleration.

### Brief description of the Drawings

These features and advantages of the invention will appear more clearly upon reading the following description, provided solely as a non-limiting example, and done in reference to the appended drawings, in which:
Figure 1 shows a top view (along a Z direction) of a wearable device according to an embodiment.
Figure 2 shows a view from below (along a Z direction) of the wearable device according to an embodiment.
Figure 3 shows a view of below (along a Z direction) of a sensors printed circuit board of the wearable device of Figure 2.
Figure 4 shows a cross sectional view along the XZ plane of the wearable device of Figure 2.
Figure 5 shows a diagram of the device with some components, especially electronic components.
Figure 6 shows views of bottom of different embodiments of sensors printed circuit boards.
Figure 7 shows a three-dimensional view of bottom of the sensors printed circuit board of Figure 3.
Figure 8 shows a view of bottom of the sensors printed circuit board of Figure 3, without a spacer.
Figure 9 shows a three-dimensional top view of the sensors printed circuit board of Figure 3.

### Detailed description

In the following description, the notions of "top" and "bottom", "horizontal" and "vertical" are defined in relation to the Z direction. The top is in the direction of a glass of the watch (or screen) and the bottom is in the direction of a case back, in contact with the wrist, as they will be described below. The vertical direction is along the Z direction and any horizontal direction is orthogonal to the Z direction.

### GENERAL DESCRIPTION

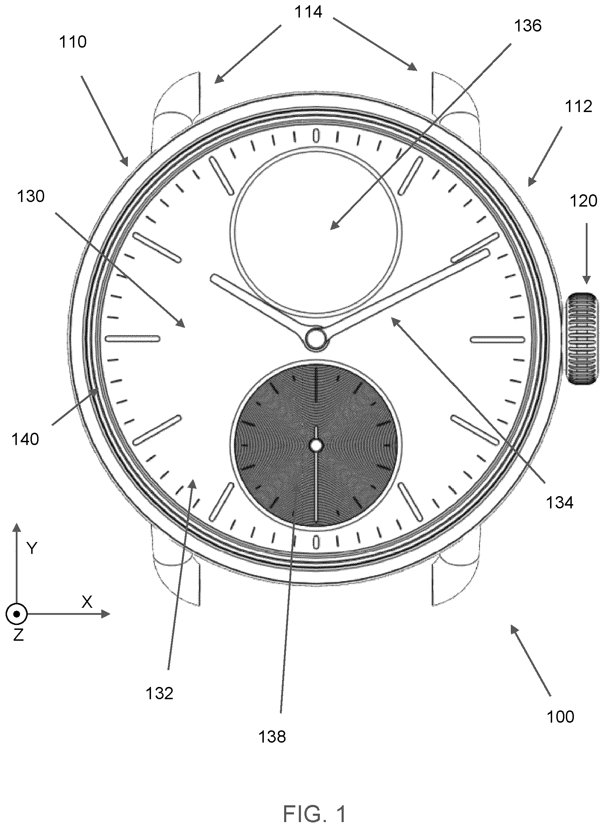

A wearable device 100 (also referred as "the device 100" in the following description) is represented on Figure 1. The device 100 is configured to be positioned on a user's wrist. In a particular embodiment, which is the one illustrated, the device is a watch. The watch may comprise a wristband. Nevertheless, in the context of the present description, the term watch does not necessarily include the wristband, which is generally manufactured elsewhere and may be assembled at points of sale. The wearable device 100 could also be an activity tracker for example.

In particular, Figure 1 illustrates an electronic watch 100, of the hybrid type with a dial, mechanical hands, and possibly a display integrated into the dial. A standard reference mark (XYZ) is shown in these figures.

In a non-represented variant, the watch may be a non-hybrid watch without mechanical hands but with a display.

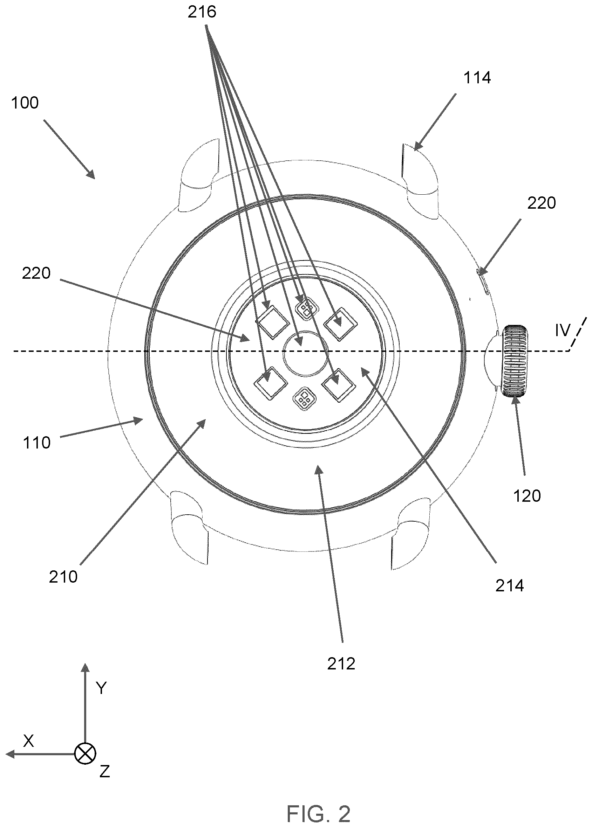

The watch 100 may comprise an enclosure 110 and a case back 210 shown on Figure 2.

When the device 100 is a watch, the enclosure may also be referred to as a watchcase. The enclosure 110 defines an internal volume suitable for receiving various components, such as electronic components, as it will be explained more in details below. The enclosure 110 protects the internal volume from notably dust, water, dampness, and shocks. The enclosure 110 may include a lateral wall 112, which is generally visible when the watch 100 is worn on the wrist. The enclosure 110 may include lugs 114 for attaching a wristband (not shown in the figures). In particular, the enclosure 110 may comprise two pairs of lugs 114, on both side of the enclosure 110. The enclosure 110 may be integral or be formed by a plurality of parts.

The watch 100 further comprises a crown 120 protruding through the enclosure 110. As visible on Figures 1 to 3, the crown 120 protrudes orthogonally to the Z axis, along an X axis. In the illustrated embodiments, the device 100 comprises a unique crown 120. In a non-represented embodiment, the device 100 may comprise a plurality of crowns 120.

The crown 120 is a hardware input for the user. In particular, the crown 120 may be used by the user to set the time, to navigate in the menu displayed on the display 136, to launch the recording of an activity, etc. The crown 120 may a push-button and/or a rotary wheel. In the illustrated embodiment, the crown 120 is a push-button and a rotary wheel, i.e. the crown 120 may be rotated around a crown axis A, parallel to the X axis and may be translated along the crown axis A.

The case back 210 is the back side of the watch 100. The case back 210 is configured to be at least partially in contact with the skin of the user's wrist. The case back 210 may comprise a surrounding member 212 (e.g., an annular member) and a lens 214, such as a glass lens, which interfaces with the skin of the wrist. The surrounding member 212 may surround the lens 214.

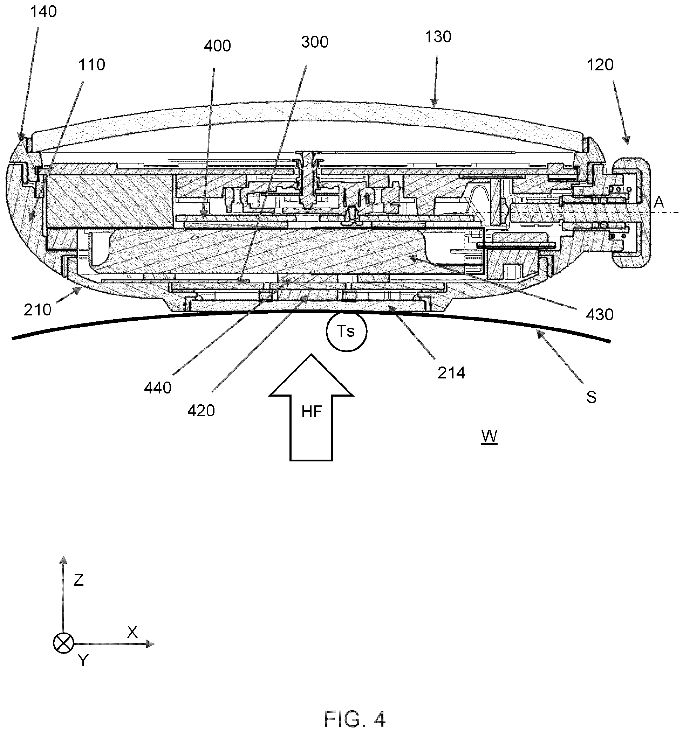

The device 100 comprises physiological sensors 216 configured to measure physiological data of the user. For example, the physiological sensors 216 may include an optical sensor. As illustrated on Figure 4, the physiological sensors 216 are positioned behind the lens 214 and will be described more in detail later.

In one embodiment illustrated on Figures 2 and 4, the enclosure 110 and the case back 210 are two separate pieces. In this embodiment, the enclosure 110 and the case back 210 may be separated by a seal. Alternatively, the enclosure 110 and the case back 210 are integral with each other. In this embodiment, the enclosure 110 and the case back 210 are a single mechanical piece.

The watch 100 may further include a glass 130, typically mounted on the enclosure 110, such that the glass 130 is maintained. The glass 130 may comprise a typically transparent protective glass and may be made of glass, ceramic, plastic or any transparent material. The outline of the glass 130 is here typically circular in shape.

In the case of a hybrid watch, below the glass 130, the watch 100 further comprises a dial 132 with physical hands 134. The dial 132 may further accommodate a display 136 (e.g., with an opening in the dial that allows a display positioned just below the dial to be visible), which occupies, for example, a small space below or within the dial 132. The watch 100 may further comprise an additional dial 138 for displaying for example the daily number of steps made by the user or another physical activity amount indicator. The glass 130 protects these parts and allows them to be seen through.

In the case of an Apple Watch^{™}-like "smartwatch", non-represented on the Figures, under the glass 130, the watch 100 comprises a screen that occupies a width close to the width of the watch 100. In one embodiment, the screen may display hands. The glass 130 is then the protective glass of the display.

The watch 100 may further comprise a bezel 140, mounted on the enclosure 110. The bezel 140 is positioned around the glass 130 (radially external to the glass around the Z direction). In the illustrated embodiments, the bezel 140 is mounted in a fixed way with respect to the enclosure 110. In a non-represented embodiment, the bezel 140 may be rotatably mounted with respect to the enclosure 110, to form a "diver type" watch for example.

### PCB

The device 100 may comprise at least a printed circuit board (also called "PCB"). Each PCB is a medium connecting different electronic components together in a circuit. Each PCB includes a thin rigid board containing a printed circuit.

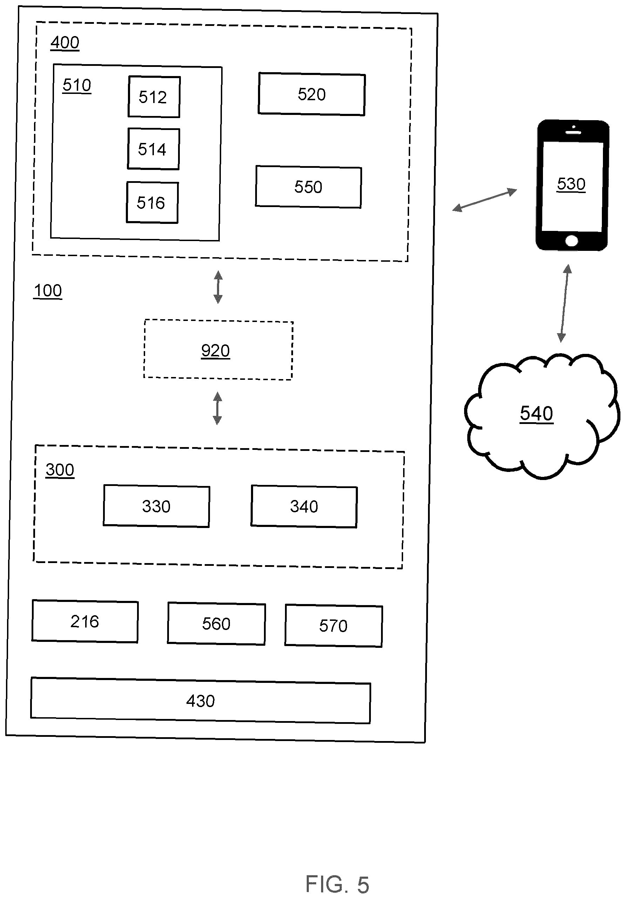

In particular, the device 100 may comprise a main printed circuit board 400 (also called "main PCB"), visible on Figure4 and 5 (schematically in dotted lines). The main PCB 400comprises a main control unit 510, schematically illustrated on Figure 5.

The control unit 510 is used to control at least a part of the on-board electronics of the device 100. The control unit 510 of the main PCB may be configured to control the battery, the display on the screen, sensors, etc.

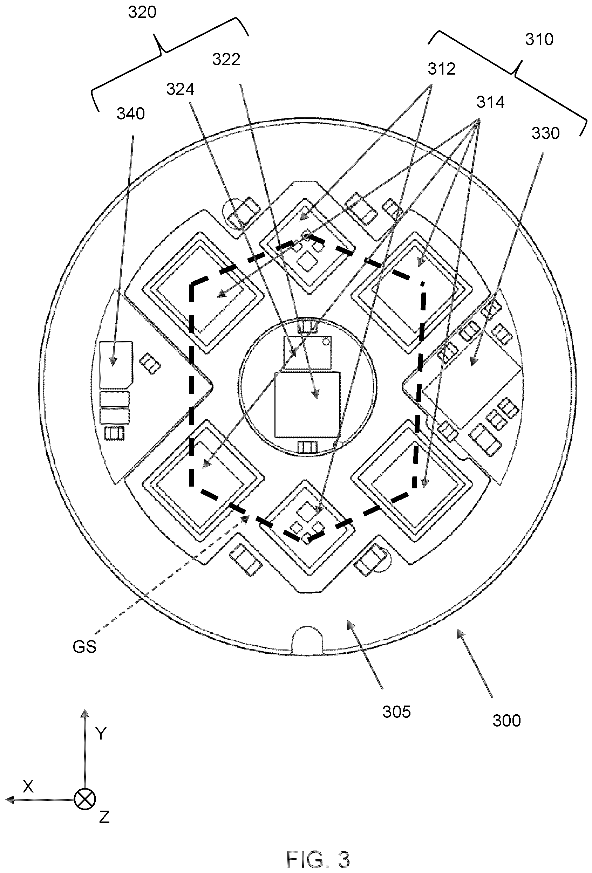

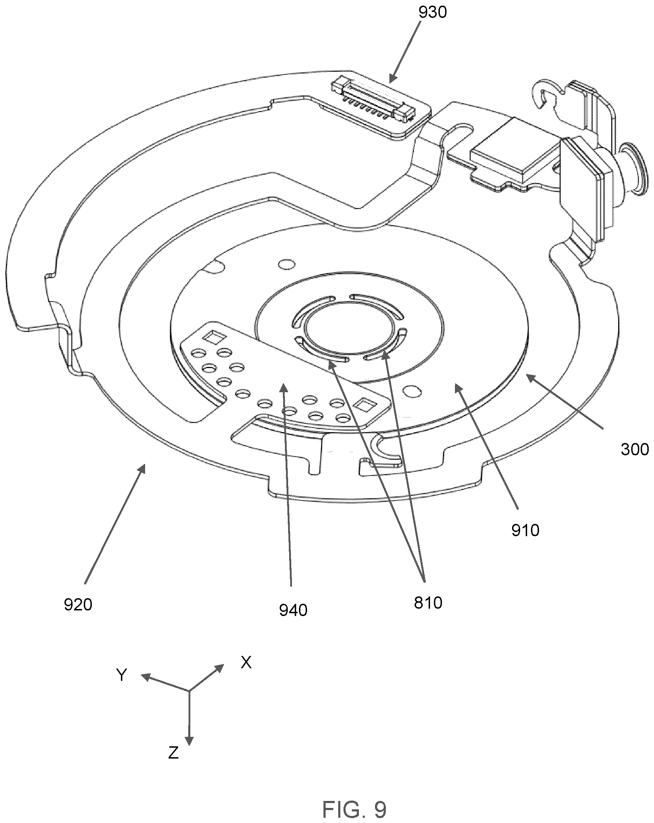

The device 100 may comprise a sensors printed circuit board 300 (also called "sensors PCB" or "opto PCB"), visible on Figures 3-4, 5 (schematically in dotted lines), and 6-9 comprising a topside 910 (visible on Figure 9) and a backside 305 (visible on Figure 3). The topside 910 is oriented towards the glass 130 and the backside 305 is oriented towards the case back 210 (or the lens 214). The physiological sensors 216 are attached to the backside 305 of the sensors PCB 300, as it will be described more in detail later. Other components may also be mounted on the sensors PCB 300 as it will be explained below.

The main PCB 400 may be connected to the sensors printed circuit board 300 through a flexible printed circuit 920 (also called "FPC"), visible on Figure 9. In particular, the flexible printed circuit 920 comprises two connectors 930, 940 to connect the flexible printed circuit respectively to the main PCB 400 and to the sensors printed circuit board 300.

### CONNECTIVITY

The device 100 includes a control unit 510, including a processor 512, a memory 514 and an I/O interface 516 to communicate with other components of the device 100. The processor 512 is configured to execute instructions stored in the memory 514.

In reference to Figure 5, the device 100 comprises a wireless communication module 520, such as a Bluetooth or Bluetooth Low Energy module or a Wi-Fi module or a cellular module (GSM, 2G, 3G,4G, 5G, Sigfox, etc.), which allows it to communicate bidirectionally with at least one external terminal 530, such as a smartphone. The external terminal 530 may then communicate (bidirectionally) with a remote server 540 for data storage and processing. Alternatively or additionally, the wireless communication module 520 may communicate directly with the remote server 540, such as via the cellular network or via a Wi-Fi network. Data obtained by the device 100, such as an electrocardiogram, but also indications of heart rate, activity or oxygen saturation, are transmitted to the external terminal 530 via the wireless communication module 520. The control unit 510 may process certain signals before sending them, to limit the size of the transmitted data.

### ECG

The device 100 may be configured to measure an electrocardiogram (also called "ECG") of the user.

To retrieve electrical signals generated by the human body, the device 100 comprises an ECG sensor. In particular, the ECG sensor comprises a set of electrodes (referred to as ECG electrodes) and an ECG electronic module 550, to which the ECG electrodes are electrically connected. By electrode is meant a conductive part capable of receiving an electric current or voltage. The part may be made of a conductive material or comprise a conductive coating. By "conductor" is meant "electrically conductive". The ECG module 550 may be mounted on the main PCB 400.

In the illustrated embodiment, the device 100 comprises only two ECG electrodes. In a non-represented embodiment, the device 100 may comprise a third ECG electrode, for example arranged on the case back 210.

In reference to Figure 3, the first ECG electrode is located on the case back 210 so as to be in contact with the skin of the user's wrist on which the user is wearing the watch 100. The first ECG electrode is electrically connected to the ECG module 550. In one embodiment, the case back 210 is the first ECG electrode. In that embodiment, the case back 210 is made of an electrically conductive material, such as metal.

In a variant, the first ECG electrode in arranged on the lens 214, for example with a metallic coating.

The second ECG electrode may be arranged on the bezel 140, such that the user may touch any portion of the bezel 140 to perform an ECG. Any portion of the bezel 140 means any portion of the surface of the bezel accessible to a user by touching. In this embodiment, the crown 120 is not an ECG electrode.

In a variant, the second ECG electrode is arranged on the crown 120.

The two electrodes are electrically connected to the ECG module 550. The ECG module 550 is configured for example to impose a potential on one of the two ECG electrodes and to measure a potential of the other of the two ECG electrodes.

The ECG module 550 is configured to retrieve electrical signals from the human body and to, after processing, generate an electrocardiogram signal or a datum representative of information about the electrocardiogram.

### ACCELEROMETER

The device 100 may further comprise an accelerometer 560. The accelerometer is an electromechanical sensor that measures acceleration forces. These forces can be static, such as the constant force of gravitation, or dynamic when the device 100 moves or vibrates. The accelerometer forces may be used notably for tracking sleep, activity, etc.

### ALTIMETER

The device 100 may further comprise an altimeter 570. The altimeter may be a barometer configured to measure the pressure of the air in a cavity fluidically connected with the outside through openings 220 in the enclosure 110 visible on Figure 2.

### PHYSIOLOGICAL SENSORS

The physiological sensors 216 are arranged at the case back 210, so that they may interact with the user's body. The first ECG electrode may at least partially surround the physiological sensor 216.

The physiological sensors 216 may comprise an optical sensor 310 and a heat flux sensor 322. The physiological sensors 216 may further comprise a skin temperature sensor 324. The heat flux sensor 322 and the skin temperature sensor 324 may be part of a temperature unit 320.

### Optical sensor 310

The optical sensor 310 comprises several optical components, in particular at least one light emission assembly 312 to emit light and at least one light reception assembly 314 to receive light emitted from the at least one light emission assembly 312, and more particularly light that went through the user's wrist W. In other words, each of the at least one light emission assembly of the optical sensor is configured to emit light towards each of the at least one light reception assembly of the same optical sensor. In other words, each of the at least one light reception assembly of the optical sensor is configured to receive light from each of the at least one light emission assembly of the same optical sensor. It will be appreciated that each of the at least one light emission assembly and each of the at least one light reception assembly of the optical sensor collectively work together to provide one or more data outputted by the optical sensor. In an embodiment, the one or more data include measurement of the user heart rate and/or the saturation in oxygen of the user blood.

In a preferred embodiment, the optical sensor 310 comprises several light emission assemblies 312 and several light reception assemblies 314. Advantageously, the optical sensor 310 comprises two several light reception assemblies 314 for each light emission assemblies 312.

The distance between each light emission assembly 312 and at least one of the light reception assemblies 314 of the optical sensor, especially configured to receive red and infrared light rays, may be comprised between 8 mm and 9 mm, for example around 8.5mm. The distance between each light emission assembly 312 and at least one other of the light reception assemblies 314, especially configured to receive green light rays may be comprised between 3 mm and 4 mm, for example around 3.5mm. The term "around" is used herein to indicate variations equal to or less than 5%.

The at least one light emission assembly 312 and the at least one light reception assembly 314 may be mounted on the backside 305 of the sensors PCB 300. Their arrangement will be described more in detail later.

The light emission assembly 312 may comprise one or more LEDs, such as a green LED, a red LED and at least an infrared LED, and/or one or more other sources of light. The light emission assembly 312 may comprise preferably two infrared LED, at two different wavelengths, for example 850 nm and 940 nm. As visible on Figure 3, each light emission assembly 312 may comprise four LEDs, which are schematically represented by squares in each light emission assembly 312 of Figure 3.

The light reception assembly 314 may be a photoreceptor, such as a photodiode.

The optical sensor 310 may further comprise an optical module 330. The optical module 330 is configured to generate the instructions for the at least one light emission assembly 312 to emit light and to receive electrical signals from the at least one light reception assembly 314. The optical module 330 may be configured to determine a heart rate of the user based on the received optical signals. Typically, the optical module 330 includes an ADC (analog to digital converter) and a processor.

The optical module 330 may be mounted on the backside 305 of the sensors PCB 300.

### Temperature unit 320

Referring to Figure 4, the heat flux sensor 322 is configured to measure a heat flux HF coming from the wrist W of the user. In particular, the heat flux HF occurs from the body of the user, across the skin S, crossing the lens 214 and reaches the heat flux sensor 322.

The heat flux sensor 322 is integral, meaning that the different components of the heat flux sensor 322 are not spaced apart in the device 100 (and therefore not made of two independent temperature sensors spaced apart and used to compute a temperature gradient therebetween). The heat flux sensor 322 may be in the form of a sandwich-like structure comprising multiple layers of different materials. In particular, the heat flux sensor 322 may be as described in documents EP2938980 and WO2016128247. The smallest dimensions of the heat flux sensor 322 in a XY plane may be between 1.8 mm and 4 mm, even between 1.9 and 3 mm, which is usually bulkier than the biggest dimension of a temperature sensor in a XY plane. In one embodiment, the heat flux sensor 322 is larger than a light emission assembly 312 in a XY plane.

The skin temperature sensor 324 is configured to determine a skin temperature Ts of the user. The skin temperature sensor 324 may be a thermistor sensor. In a variant, the skin temperature sensor 324 may be an infrared thermal sensor. The skin temperature sensor 324 is less bulky than the heat flux sensor 322 and therefore easier to integrate in the device 100.

The heat flux sensor 322 and the skin temperature sensor 324 may be arranged next to one another, with a gap therebetween. The heat flux sensor 322 and the skin temperature sensor 324 may be attached onto the same side of the sensors PCB 300. In particular, the heat flux sensor 322 and the skin temperature sensor 324 may be mounted on the backside 305 of the sensors PCB 300. Their arrangement will be described more in detail later.

The temperature unit 320 may further comprise a core body temperature module 340 configured to receive data from the heat flux sensor 322 and advantageously the skin temperature sensor 324 to determine a core body temperature of the user. The core body temperature module 340 may be mounted on the backside 305 of the sensors PCB 300.

Core body temperature refers to the temperature of the user body's internal organs, such as the heart, liver, brain and blood. Core body temperature is different from the skin temperature which is measured at the outermost surface of the body. Because the skin is more exposed to the environment, skin temperature fluctuates more widely than body core temperature. Skin is likely to cool due to sweat and evaporation, for instance, as the body works to bring down the core body temperature. Skin temperature can be significantly lower than the body core temperature in the limbs and extremities for example.

The core body temperature module 340 is configured to collect the data from the heat flux sensor 322 and advantageously the skin temperature sensor 324 at a frequency comprised between 0.1 Hz and 0.5 Hz, notably 0.2 Hz.

The core body temperature module 340 may also be configured to collect the heart rate determined by the optical module 330 and configured to determine the core body temperature of the user based at least on the heat flux and skin temperature data and on the heart rate.

The core body temperature module 340 may be configured to collect the acceleration signals delivered by the accelerometer 560 and configured to determine the core body temperature of the user based at least on the heat flux and skin temperature data and on the acceleration signals.

### SENSORS ARRANGEMENT

As explained before, the light emission assembly 312, the light reception assembly 314 and the heat flux sensor 320 are attached to the backside 305 of the sensors PCB 300 and positioned behind the lens 214 which interfaces with the skin S of the wrist W, as visible of Figure 4. When the device 100 comprises a skin temperature sensor 324, the skin temperature sensor 324 is also attached to the backside 305 of the sensors PCB 300 and positioned behind the lens 214.The temperature module 340 and the optical module 330 are also attached to the backside 305 of the sensors PCB 300.

There is provided an opaque mask 220 on the lens 214 (e.g., an inner wall of the lens 214). In particular, the mask 220 may be deposited on the lens 214 and then etched to remove matter at prescribed locations where the light rays are emitted on purpose and received on purpose for the PPG function. The opaque mask 220 enables to reduce optical crosstalk since no light can enter the inner space of the device from outside at positions others than the light reception assemblies 314. The opaque mask 220 may cover the temperature unit 320, so that the temperature unit 320 may not be visible by the user through the lens 214.

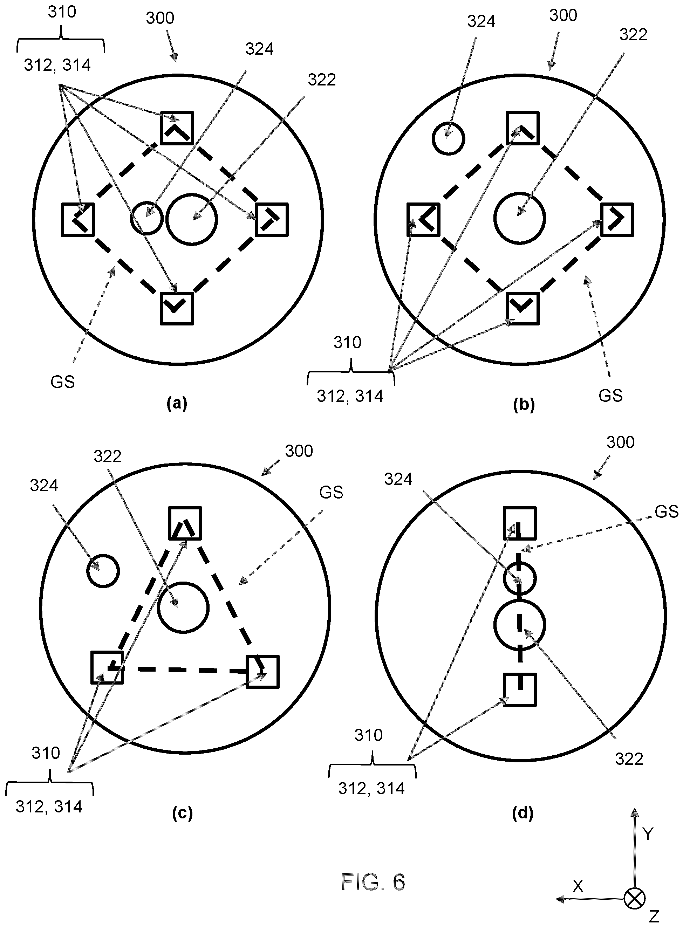

In reference to Figures 3 and 6, positions of the at least one light emission assembly 312 and the at least one light reception assembly 314 of the same optical sensor 310 form a geometrical shape GS on the case back 210 (lens 214 here), as orthogonally projected on the case back 210 (on lens 214 here). In other words, in a transversal plane XY, the positions of the at least one light emission assembly 312 and the at least one light reception assembly 314, projected orthogonally on the case back 210, form the geometrical shape GS. The geometrical shape GS is defined by the respective geometrical centers of the at least one light emission assembly 312 and the at least one light reception assembly 314 of the same optical sensor 310. When the optical sensor 310 comprises at least three optical components, the geometrical shape GS is in particular a convex shape.

When the case back 210 (the lens 214 here) is flat along the XY plane, an orthogonal projection is a projection according to Z. When the case back is slightly curved, the orthogonal projection is a projection orthogonal to a tangential plane of the case back 210, (which is essentially a projection according to axis Z if the curvature is slight).

In one embodiment, the at least one light emission assembly 312 and the at least one light reception assembly 314 of the same optical sensor 310 are positioned in a same transversal plane XY (behind the case back 210 or the lens 214), called sensor plane. This is for example achieved using the backside 305 of the sensors PCB 300, to which all those components are attached.

The heat flux sensor 324 is arranged within the geometrical shape GS when orthogonally projected on the case back 210 (on lens 214 here) as visible on Figures 3 and 6. In other words, the position of the center of the heat flux sensor 324 projected on the case back 210 is within the interior area formed by the geometrical shape GS. When the geometrical shape GS is a segment, the segment crosses the heat flux sensor 324.

In one embodiment, the heat flux sensor 324 is also positioned in the sensor plane. This is for instance achieved using the backside 305 of the sensors PCB 300, to which the heat flux sensor 324 is attached. In this embodiment, all the brackets "as orthogonally projected on the case back 210" may also be replaced by "in the sensors plane". At least a light emission assembly 312 and at least a light reception assembly 314 may be arranged on either side of the heat flux sensor 322 (as orthogonally projected on the case back 210). Therefore, at least a light path starts from one of the light emission assemblies 312, passes underneath the heat flux sensor 322 and reaches one of the light reception assemblies 314.

In a preferred embodiment, the optical sensor 310 comprises several light emission assemblies 312 and several light reception assemblies 314. The formed geometrical shape GS is then a polygon. The heat flux sensor 322 is within the polygon (as orthogonally projected on the case back 210).

The skin temperature sensor 324 may be also located within the geometrical shape GS (as orthogonally projected on the case back 210). In a variant, the skin temperature sensor 324 may be also located outside the geometrical shape GS (as orthogonally projected on the case back 210).

In a preferred embodiment, the heat flux sensor 322 (e.g., at least a part of it or its own geometrical center) is arranged at the geometrical center of the geometrical shape GS (as orthogonally projected on the case back 210). This may include an area of 25% of the area of the geometrical shape GS and including said geometrical center or, alternatively, may strictly mean the center. For example, if the geometrical shape GS is a square, the heat flux sensor 322 (e.g., at least a part of it or its centroid) is arranged at the intersection of the two diagonals of the square. Advantageously, the heat flux sensor 322 is arranged at the center of the lens 214. Advantageously, the heat flux sensor 322 is arranged at the middle of the case back 210.

This arrangement enables to obtain a device 100 that meets the high layout constraints for the optical components. In particular, the geometrical shape enables to obtain a short light path within the wrist tissue especially for the green measurements and a long light path within the wrist tissue especially for the red and infrared measurements. By placing the heat flux sensor 322 within the geometrical shape, the heat flux sensor sensitivity is improved. Indeed, the contact between the heat flux sensor 322 and the skin S of the wrist W is improved and homogenized. The heat flux sensor is quite sensitive to local flux variations, and at the center these flux variations are the smallest because the skin-sensor contact is maximized. Moreover, the heat flux sensor 322 is less sensitive to the outside temperature and the measured heat flux HF is close to that of the skin S. Also, this case back arrangement enables the physiological sensors to occupy a limited spatial area on the case back 210, enabling to arrange an ECG electrode around the lens 214 which needs to contact also the wrist of the user.

### Hexagon embodiment

In reference to Figure 3, the optical sensor 310 may comprises two light emission assemblies 312 and four light reception assemblies 414. The two light emission assemblies 312 and four light reception assemblies 314 collectively work together to provide one or more data outputted by the optical sensor 310. For example, each LED of each of the two light emission assemblies 312 emits light towards each of the four light reception assemblies 314. In this arrangement, the formed geometrical shape GS is then a hexagon (e.g. a semi-regular as illustrated on Figure 3 or a regular hexagon). The heat flux sensor 322 is within the hexagon. In particular, in an embodiment, the heat flux sensor 322 is within an area or occupies an area of the geometrical shape GS that is surrounded by light rays that are emitted by each LED of the two light emission assemblies 312 towards each of the four light reception assemblies. In an embodiment, this area can be at least partly traversed by one or more light rays emitted by LEDs of the two light emission assemblies 312. It was found that this unconventional arrangement of the optical sensor 310 and heat flux sensor 322 is greatly beneficial to improve sensitivity of the heat flux sensor 322. Preferably, the skin temperature sensor 324 is also within the hexagon (as orthogonally projected on the case back 210).

The four light reception assemblies 314 form a rhombus, preferably a square centered on the center of the case back 210. Each light emission assembly 312 is arranged between two light reception assemblies 314 (as orthogonally projected on the case back 210). Preferably the two light emission assemblies 312 are placed on either side of the heat flux sensor 322 (as orthogonally projected on the case back 210).

The optical module 330 and the core body temperature module 340 may be located outside the geometrical shape GS (as orthogonally projected on the case back 210). Preferably, the optical module 330 and the core body temperature module 340 are placed on either side of the heat flux sensor 322.

### Alternative embodiments

Several variations of arrangements of the physiological sensors 216 are represented on Figure 6. In a similar manner as the arrangement of Figure 3, each light emission assembly 312 and each light reception assembly 314 of the optical sensor 310 of Figures 6(a)-(d) collectively work together to provide one or more data outputted by the optical sensor 310. Moreover, each LED of each light emission assembly 312 is configured to emit light towards each light reception assembly 314 of the optical sensor. The heat flux sensor and optionally the skin temperature sensor of the arrangements of Figures 6(a)-(d) is/are arranged within the geometrical shape GS formed by the one or more light emission assemblies 312 and the one or more light reception assemblies 314 of the optical sensor 310. In the arrangements of Figures 6(a)-(d), the heat flux sensor and optionally the skin temperature sensor may be within an area or may occupy an area of the geometrical shape GS that is surrounded by light rays that are emitted by each LED of the one or more light emission assemblies 312 towards each of the one or more light reception assemblies 314.

In reference to Figure 6 (a) and (b), the optical sensor 310 may comprise four optical components, for example two light emission assemblies 312 and two light reception assemblies 314. In a variant, the optical sensor 310 may comprise one light emission assemblies 312 and three light reception assemblies 314. The formed geometrical shape GS is then a rhombus, preferably a square (as orthogonally projected on the case back 210). The heat flux sensor 322 is within the rhombus (as orthogonally projected on the case back 210). As visible on Figure 6 (a), the skin temperature sensor 324 may be also within the rhombus (as orthogonally projected on the case back 210). In a variant represented on Figure 6 (b), the skin temperature sensor 324 may be arranged outside the rhombus (as orthogonally projected on the case back 210).

In reference to Figure 6 (c), the optical sensor 310 may comprise three optical components, for example one light emission assembly 312 and two light reception assemblies 314. The formed geometrical shape GS is then a triangle (as orthogonally projected on the case back 210). The heat flux sensor 322 is within the triangle (as orthogonally projected on the case back 210). The skin temperature sensor 324 may be also within the triangle. In a variant represented on Figure 6 (c), the skin temperature sensor 324 may be arranged outside the triangle (as orthogonally projected on the case back 210).

In reference to Figure 6 (d), the optical sensor 310 may comprise one light emission assembly 312 and one light reception assembly 314. The formed geometrical shape is then a segment (as orthogonally projected on the case back 210). The heat flux sensor 322 is arranged on the segment (as orthogonally projected on the case back 210). As represented on Figure 6 (d), the skin temperature sensor 324 may be also arranged on the segment. In a variant, non represented, the skin temperature sensor 324 may be arranged outside the segment (as orthogonally projected on the case back 210).

### INSULATING SLOTS

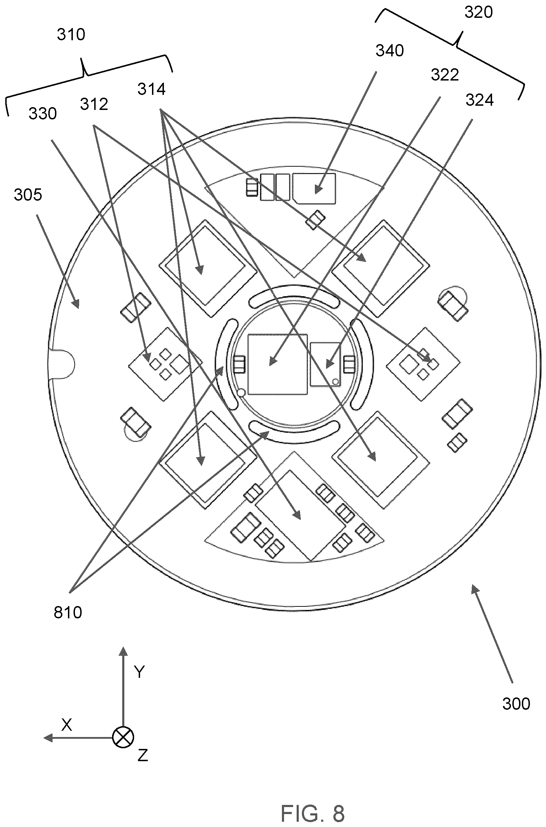

In reference to Figure 8, the sensors PCB 300 may comprise at least an insulating opening, such as a slot 810 filled with a thermal insulator. The thermal insulator is in particular air. The at least insulating slot 810 is arranged between the heat flux sensor 322 and the optical sensor 310, notably between the heat flux sensor 322 and the at least light emission assembly 312.

The insulating slots 810 enable to thermally insulate the heat flux sensor 322 from the optical sensor 310 which may produce some heat when working and which could interfere with the measure of the heat flux HF coming from the wrist W of the user. As indicated before, the location of the heat flux sensor 322 within the geometrical shape GS enables to homogenize the influence of the produced heat by the optical sensor 310 on the heat flux sensor 322.

Each insulating slot 810 is preferably a pass-through opening. In other words, each slot is open both on the topside 910 and the backside 305 of the sensors PCB 300.

As visible on Figure 8, the sensors PCB 300 may comprise several insulating slots 810, for example regularly located (such as a circle), and notably four insulating slots 810. Each insulating slot 810 may form a portion of a circle, preferably centered on the center of the case back 210 and/or centered on the center of the lens 214.

The width of each insulating slot 810 is comprised between 0.1 mm and 1 mm, preferably between 0.3 mm and 0.7 mm.

The insulating slots 810 are arranged all around the heat flux sensor 322, and, if located nearby, the skin temperature sensor 324. In particular, as visible on Figure 8, at least on insulating slot 810 is arranged between the heat flux sensor 322 and each light emission assembly 312. At least one insulating slot 810 is arranged between the heat flux sensor 322 and the optical module 330. At least one insulating slot 810 is arranged between the heat flux sensor 322 and the core body temperature module 340.

### SPACER

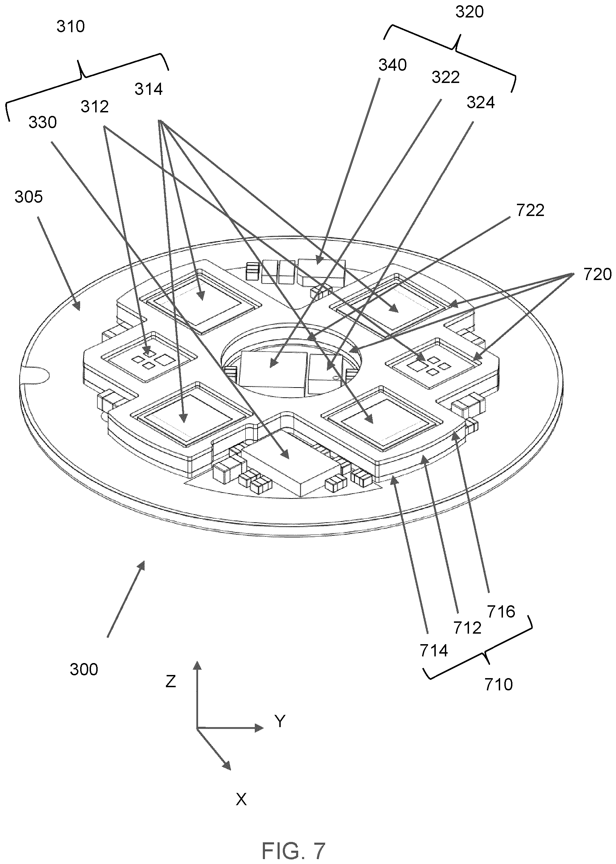

In reference to Figure 7, the device 100 may comprise at least one spacer 710. The spacer 710 comprises a plurality of cavities 720 in which the temperature unit 320 and the optical sensor 310 are arranged. In particular, the spacer 710 is arranged between the sensors PCB 300 and the case back 210 (here the lens 214). The spacer 710 is therefore arrange at (or includes) the sensor plane.

The spacer 710 forms a receptacle for the physiological sensors 216. The spacer 710 also enables to optically isolate the light emission assemblies 312 from the light reception assemblies 314. The spacer 710 enables therefore to functionally complement the above mentioned opaque mask 220, to avoid any undesired optical leakage from the light source. Thereby the light reception assemblies 314, when the wristwatch is worn at wrist, only receive perfused light through the wrist W.

The spacer 710 comprises at least two layers comprising a rigid layer 712 and a compressible layer, preferably two compressible layers 714, 716 on each side of the rigid layer 712. The compressible layers 714, 716 may be foam layers. The rigid layer 712 forms a rigid frame of the spacer 710 and the compressible layers enable to absorb mechanical tolerances between the sensors PCB 300 and the lens 214.

Each of the at least one light emission assembly 312, the at least one light reception assembly 314 and the heat flux sensor 322 are arranged in respective cavities 720 of the plurality of cavities. One of the cavities 720 is a central cavity 722, located centrally on the case back 210, as projected orthogonally on the case back 210, with wider dimensions than the heat flux sensor 322. The central cavity 722 is configured to accommodate the heat flux sensor 322. In one embodiment, the skin temperature sensor 324 is arranged in the same central cavity 722 as the heat flux sensor 322. The central cavity 722 is preferably circular.

The device 100 may comprise a thermal conductive gap filler 420 arranged in the central cavity 722. The thermal conductive gap filler 420 is made from a thermally conductive material and partially surrounds the heat flux sensor 322, and, if applicable, the skin temperature sensor 324. The conductivity of the thermal conductive gap filler 420 is for example comprised between 0.3 W/mK and 10 W/mK, preferably between 0.8 W/mK and 3 W/mK. The thermally conductive material is a for example a silicone elastomer.

The thermal conductive gap filler 420 is compressed between the sensors PCB 300 and the lens 214. The thermal conductive gap filler 420 fills preferably all the space of the central cavity 722.

The thermal conductive gap filler 420 enables to homogenize the heat flux HF coming from the wrist and crossing the lens 214, and thus minimize parasitic heat flows.

### BATTERY

The device 100 further comprises a battery 430 configured to provide energy for the device 100, and in particular for the electronic components of the device 100. The battery 430 is preferably rechargeable.

A rechargeable battery 430 is configured to store chemical energy and to convert this chemical energy into electrical energy. The battery 430 is configured to be recharged a plurality of times, i.e. reversing the electrochemical reaction to recharge the chemical energy stored into the battery 430. To that end, the device 100 is configured to cooperate with an electrical charger.

The battery 430 may be for example a Lithium Ion battery or a Lithium Polymer battery. The battery 430 has may have a button shape but may also present a parallelepiped shape.

As visible on Figure 4, the battery 430 may be arranged next to the topside 910 of the sensors PCB 300, for example between the main PCB 400 and the sensors PCB 300.

In an embodiment, the device 100 comprises a thermal exhaust 440 made from a thermally conductive material and arranged on the topside 910 of the sensors PCB 300. The thermally conductive material is for example a silicone elastomer, notably the same as for the gap filer 420.

The thermal exhaust 440 is arranged between the sensors PCB 300 and the battery 430.

The thermal exhaust 440 enables to dissipate the heat from the sensors PCB 300 to the battery 430, which forms a large metallic mass/surface. The battery 430 may comprise a surrounding metallic film, enhancing the battery's thermal conductivity.

## Claims

1. A wearable device (100) configured to be positioned on a user's wrist (W), the wearable device (100) comprising:
- a case back (210) configured to be at least partially in contact with the user's wrist (W),
- a heat flux sensor (322), configured to measure a heat flux (HF) coming from the user's wrist (W),
- an optical sensor (310) comprising at least one light emission assembly (312) to emit light and at least one light reception assembly (314) to receive light from the light emission assembly (312),
wherein positions of the at least one light emission assembly (312) and the at least one light reception assembly (314) form a geometrical shape (GS) on the case back (210), as projected orthogonally on the case back (210),
wherein the heat flux sensor (322) is arranged within the geometrical shape (GS), as projected orthogonally on the case back (210).

2. The device (100) according to claim 1, wherein the heat flux sensor (322) is arranged at a center of the geometrical shape (GS).

3. The device (100) according to claim 1 or 2, wherein the device (100) further comprises a skin temperature sensor (324), to determine a skin temperature of the user,
wherein the skin temperature sensor (324) is also located within the geometrical shape (GS), as projected orthogonally on the case back (210).

4. The device (100) according to any of claims 1 to 3, wherein at least a light emission assembly (312) and at least a light reception assembly (314) are disposed on either side of the heat flux sensor (322), as projected orthogonally on the case back (210).

5. The device (100) according to any of claims 1 to 4, wherein the optical sensor (310) comprises a plurality of light emission assemblies (312) and a plurality of light reception assemblies (314) that are collectively arranged to form the geometrical shape, the formed geometrical shape (GS) being a polygon, the heat flux sensor (322) being within the polygon.

6. The device (100) according to any of claims 1 to 5, wherein the optical sensor (310) comprises two light emission assemblies (312) and four light reception assemblies (314) that are collectively arranged to form the geometrical shape, the formed geometrical shape (GS) being a hexagon.

7. The device according to any of claims 1 to 6, wherein the device (100) further comprises a sensors printed circuit board (300) comprising a topside (910) and a backside (305), the backside being oriented towards the case back (210), wherein the heat flux sensor (322), the at least one light emission assembly (312) and the at least one light reception assembly (314) are attached to the backside (305) of the sensors printed circuit board (300).

8. The device (100) according to any of claims 1 to 7, wherein the optical sensor (310) comprises an optical module (330), the optical module (330) being configured to generate the instructions for the light emission assemblies (312) and to recover the electrical signals from the light reception assemblies (314).

9. The device (100) according to any of claims 1 to 8, wherein the device further comprises a core body temperature module (340) configured to receive data from the heat flux sensor (322), and advantageously form the skin temperature sensor (324), to determine a core body temperature of the user.

10. The device (100) according to any of claims 1 to 9, wherein the device (100) comprises a spacer (710) with a plurality of cavities (720), wherein the heat flux sensor (322) and the optical sensor (310) are arranged in respective cavities of the plurality of cavities (720).

11. The device (100) according to claim 10, wherein one of the cavities (720) is a central cavity (722), located centrally on the case back (210) as projected orthogonally on the case back (210), the central cavity having wider dimensions than the heat flux sensor (322), said central cavity (722) being configured to accommodate the heat flux sensor (322).

12. The device (100) according to claim 11, wherein the central cavity (722) comprises a thermal conductive gap filler (420) made from a thermally conductive material and surrounding partially the heat flux sensor (322).

13. The device (100) according to any of the preceding claims in combination with claim 7, wherein the device (100) comprises a thermal exhaust (440) made from a thermally conductive material and arranged on the topside (910) of the sensors printed circuit board (300).

14. The device (100) according to any of the preceding claims in combination with claim 7, wherein the sensors printed circuit board (300) comprises at least an insulating slot (810) filled with a thermal insulator, in particular filled with air, and arranged between the heat flux sensor (322) and the optical sensor (310).

15. The device (100) according to claim 14, wherein the at least one insulating slot (810) is/are arranged all around the heat flux sensor (322), and eventually the skin temperature sensor (324).
